# EUROPEAN PATENT SPECIFICATION

(11) **EP 3 350 309 B1**
(45) Date of publication and mention of the grant of the patent: **02.11.2022**
(21) Application number: 16794028.7
(22) Date of filing: 15.09.2016
(51) Int. Cl.: C12M 1/08, C12M 1/00, B01F 23/232

(54) **REACTION DEVICE WITH AIR-LIFT TYPE INTERNAL CIRCULATION AND FERMENTATION PROCESS USING SAID DEVICE**
REAKTIONSVORRICHTUNG MIT INTERNER LUFTHEBEZIRKULATION UND FERMENTATIONSVERFAHREN UNTER VERWENDUNG DIESER VORRICHTUNG
DISPOSITIF DE RÉACTION PRÉSENTANT UNE CIRCULATION INTERNE DE TYPE À AGITATION PNEUMATIQUE ET PROCÉDÉ DE FERMENTATION UTILISANT LEDIT DISPOSITIF

(30) Priority: 18.09.2015 IT UB20153722
(43) Date of publication of application: 25.07.2018
(73) Proprietor: Eni S.p.A., 00144 Roma (IT)
(72) Inventor: OLDANI, Fabio, I-20015 Parabiago (MI) (IT); D'ARMINIO MONFORTE, Alessandra, 20149 Milano (IT)
(74) Representative: Cernuzzi, Daniele
(86) International application number: PCT/IB2016/055482
(87) International publication number: WO 2017/046720

(56) References cited:
- CN-A- 101 955 881
- CN-U- 202 107 704
- GB-A- 1 383 432
- JP-A- S5 515 718
- MERCHUK J C ET AL: "LIQUID FLOW AND MIXING IN CONCENTRIC TUBE AIR-LIFT REACTORS", JOURNAL OF CHEMICAL TECHNOLOGY AND BIOTECHNOLOGY, WILEY, vol. 66, no. 2, 1 June 1996 (1996-06-01), pages 174-182, XP000625193, ISSN: 0268-2575, DOI: 10.1002/(SICI)1097-4660(199606)66:2<174::A ID-JCTB481>3.3.CO;2-V

## Description

The present invention relates to a reaction device with air-lift type internal circulation which may be used to carry out biphasic gas-liquid and triphasic gas-solid-liquid reactions. In particular, said device may be used for carrying out fed-batch fermentations of aerobic microorganisms, such as for example yeasts.

Furthermore, the present invention relates to a fermentation process of aerobic microorganisms, such as for example yeasts, in fed-batch, carried out in the reaction device with air-lift type internal circulation which is described and claimed.

In the present patent application, all the operating conditions mentioned in the text should be taken to be preferred conditions even if this is not explicitly stated.

For the purposes of the present explanations, the term "comprise" or "include" also encompasses the term "consist in" or "essentially consist of".

For the purposes of the present explanations, unless stated otherwise, range definitions always include the extremes.

Biphasic or triphasic reactors used in fermentations are typically bubble reactors or stirred reactors. The latter are somewhat inefficient and costly from an energy standpoint and therefore their application is limited by the scale and type of reaction (T. Ichii, I. S. Takehara, H. Konno, T. Ishida, H. Sato, A. Suzuki, K. Yamazumi; "Development of a new commercial-scale airlift fermentor for rapid growth of yeast"; J. Ferment. Bioeng.; 75; [1993]).

Fed-batch fermentations are usually carried out in stirred reactors or in bubble columns, in reactors which allow a large space for the volume change over time . Air-lift systems are used in fed-batch mode in the presence of a net draft tube (as described for example in US 2004/6746862, C.-C. Fu, W.-T. Wu, S.-Y. Lu; "Performance of airlift bioreactors with net draft tube"; Enzyme Microb. Technol.; 33; [2003] and J.-Y. Wu, W.-T. Wu; "Fed-batch culture of Saccharomyces cerevisiae in an airlift reactor with net draft tube"; Biotechnol. Prog.; 7; [1991]).

Fed-batch fermentations permit higher reaction yields (Y.-H. Changa, K.-S. Changa, C.-W. Huang, C.-L. Hsuc, H.-D. Janga; "Comparison of batch and fed-batch fermentations using corncob hydrolysate for bioethanol production"; Fuel; 97; [2012]).

Air-lift fermenters are used in large-scale fermentations and, in comparison with bubble columns, permit better mixing of the liquid phase ("Bubble Column Reactors"; W.-D. Deckwer; Wiley; 1992).

In air-lift reactors, fluid dynamic parameters directly correlate with system geometry. Parameters such as "gas holdup", velocity of the bubbles and the liquid and "mass transfer" depend not only on the chemical-physic nature of the fluids but also on the geometric ratios of the reactor. The system is often operated under the best conditions imposed by the process and any variations are limited to minor oscillations around the working point (J.C. Merchuk, N. Ladwa, A. Cameron, M. Bulmer, A. Pickett; "Concentric-tube airlift reactors: Effects of geometrical design on performance"; AlChE J.; 40; [1994]). Fermentations performed in fed-batch mode achieve greater yields since the nutrients are fed in in accordance with a determined strategy which maximises the final product, avoiding phenomena such as substrate inhibition. Oxygen consumption also varies over the various stages of an aerobic fermentation. Such variation is controlled by varying the availability of dissolved oxygen as a function of the system (rotational speed of the impeller, air flow rate and system pressure).

Fed-batch fermentations bring about an increase in system volume due to continuous charging of nutrients. The variation in volume entails a reactor which is capable of supporting growth of aerobic microorganisms with a variable volume.

The variation in volume in an air-lift reactor brings about a reduction in the performance thereof since it is operated under conditions which are further from the optimum (J.C. Merchuk, N. Ladwa, A. Cameron, M. Bulmer, A. Pickett; "Concentric-tube airlift reactors: Effects of geometrical design on performance"; AlChE J.; 40; [1994]).

GB1383432A discloses a method and an apparatus for growing a microorganism and carrying out fermentation; fermentation is carried out in an apparatus consisting essentially of an ascending head chamber, a descending head chamber, and provision for agitating the liquid medium by insufflating a stream of gas into the lower portion of the ascending head chamber. In some embodiments, the ascending head chamber is arranged concentrically inside the descending head chamber.

An object of the present invention is therefore that of providing an air-lift type reaction device with variable volume which has a specific geometry.

A further object of the present invention is that of using said air-lift type reaction device with a specific geometry for carrying out fed-batch aerobic fermentation reactions.

By carrying out a fed-batch aerobic fermentation in an air-lift reactor with variable volume, it is possible to manage the fermentation by exploiting the conditions which ensure maximum air-lift performance in conjunction with the maximum oxygen demand of the fermentation.

The Applicant has accordingly invented a reaction device with air-lift type internal circulation which includes:
- a vertical cylindrical volume;
- a vertical element positioned within said volume in such a way as to form an interspace with the walls of said volume, having a cross-section which is circular and orthogonal to the vertical axis of the element and with a variable internal diameter along said axis, said element being denoted "draft tube";
- at least one gas distributor positioned on the bottom of said device;
said device being characterised in that:
- the ratio between the diameter of the internal vertical element and the internal diameter of the cylindrical volume ranges from 0.05 to 0.5, and
- the ratio between the height of the vertical element and the height of the cylindrical volume is less than 0.5.

Further purposes and advantages of the present invention will be more apparent from the following description and the appended figures, which are provided purely by way of nonlimiting example.

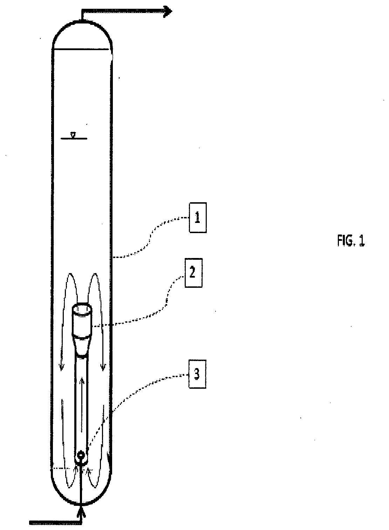

All of Figures 1-5 are preferred embodiments according to the present invention.

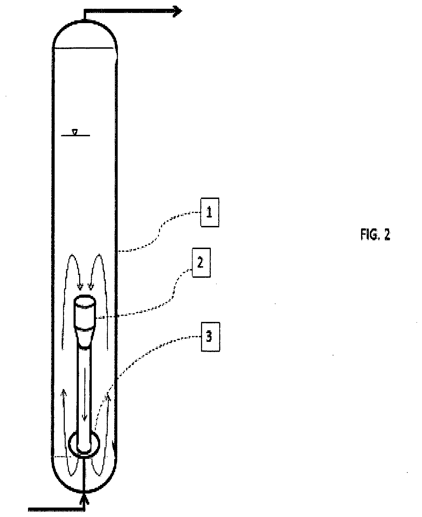

Figure 1 is an air-lift type reaction device according to the present invention, comprising a vertical element (2) and a distributor (3) for the gas phase. The gas flows through the draft tube which acts as a riser, while the cylindrical volume acts as a downcomer. Figure 2 is an air-lift type reaction device according to the present invention, comprising a vertical element (2) and a distributor (3) for the gas phase. The gas flows outside the draft tube which acts as a downcomer, while the cylindrical volume acts as a riser.

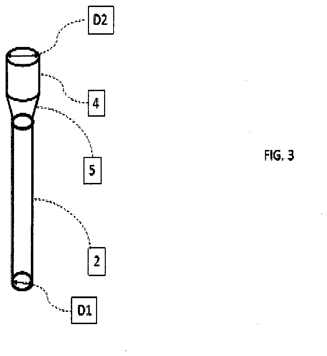

Figure 3 illustrates a specific geometry of a draft tube with a variable diameter along the axis; said draft tube comprises three parts: a cylindrical part with diameter D1 (2), a truncated cone part with diameter D1 < D2 (5) and a cylindrical part with diameter D2 (4).

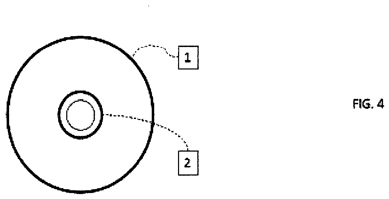

Figure 4 is a section orthogonal to the axis of an air-lift type reaction device according to the present invention. The draft tube (2) is positioned centrally relative to the axis of the outer body of the reaction device (1). According to this embodiment of the invention, the ratio between the diameter of the draft tube and the internal diameter of the cylindrical volume ranges from 0.05 to 0.5.

Figures 5, 6 and 7 illustrate the variation in liquid level within the reaction device in the course of an aerobic reaction managed in fed-batch mode.

Figure 5 illustrates the initial condition corresponding to batch mode management of the reactor during which liquid is fed into the reactor (7). Under these conditions, the level of the supernatant liquid above the draft tube (6) ranges from 0 to 1.5 times the diameter D1 of the draft tube.

Figure 6 illustrates the semi-continuous reaction mode stage during which liquid (7) is continuously fed in, raising the level in the reactor (6).

Figure 7 shows the final condition of the reaction device on completion of the reaction which corresponds to the maximum liquid level in the reactor without any continuous liquid feed.

### Detailed description.

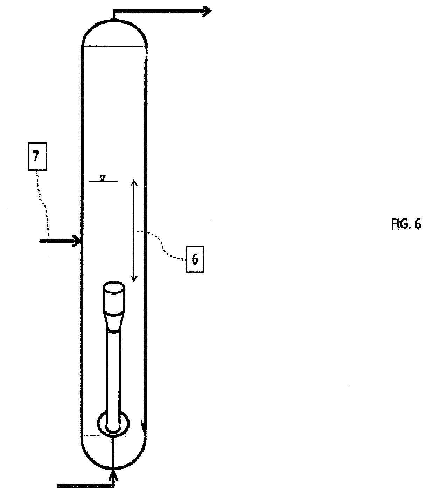

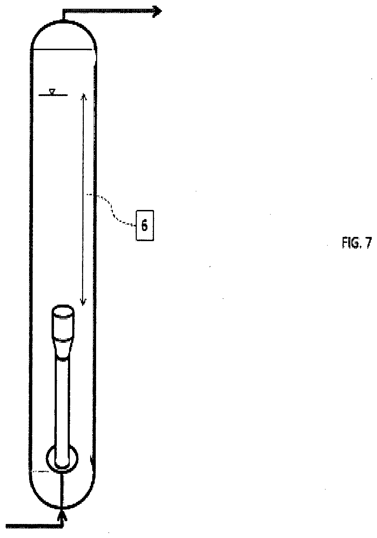

With reference to Figures 1-7, the Applicant will now provide a detailed description of the present invention.

The Applicant describes and claims a reaction device with air-lift type internal circulation which includes:
- a vertical cylindrical volume (1);
- a vertical element (2) positioned within said volume in such a way as to form an interspace with the walls of said volume, having a cross-section which is circular and orthogonal to the vertical axis of the element and with a variable internal diameter along said axis, said element being denoted "draft tube";
- at least one gas distributor (3) positioned on the bottom of said device;
said device being characterised in that:
- the ratio between the diameter of the internal vertical element (2) and the internal diameter of the cylindrical volume (1) ranges from 0.05 to 0.5, and
- the ratio between the height of the vertical element (2) and the height of the cylindrical volume (1) is less than 0.5.

The ratio between the diameter of the internal vertical element and the internal diameter of the cylindrical volume preferably ranges from 0.35 to 0.45.

The ratio between the height of the vertical element (2) and the height of the cylindrical volume (1) is preferably less than 0.5 and greater than 0.25.

The vertical element inside the reaction device is preferably formed by three bodies, two cylindrical bodies (4, 2) having different diameters and a body in the form of a truncated cone (5). Said bodies are connected to one another in such a manner that the minor base of the truncated cone (5) coincides with one of the bases of the first cylindrical body (2) and the major base of the truncated cone coincides with one of the bases of the second cylindrical body (4).

The ratio between the height of the truncated cone and the total height of the device preferably ranges from 0.03 to 0.25, more preferably between 0.04 and 0.08.

The inclination of the apothem of the truncated cone body in the draft tube preferably ranges from 15° to 30°, more preferably between 15° and 20°.

Said geometry of the draft tubes allows the liquid to recirculate more quickly in the interior thereof, ensuring better mixing of the system.

According to the present invention, the draft tube is a tubular element which, positioned inside a reaction device, divides said device into two distinct zones by creating an interspace between the tubular element and the walls of the device itself.

With reference to Figure 1, during a gas-liquid or gas-solid-liquid reaction, a stream of gas introduced by way of appropriate distributors (3) into a reaction device (1) is segregated from the liquid or liquid-solid phase thanks to the tubular draft tube element (2).

When the gas is fed in, zones of differing density are formed within the reaction device due to the presence of the draft tube: a lower density zone where the gas is more concentrated and a higher zone where it is more dispersed. This difference in density initiates circulation of the liquid phase which tends to move upwards in the zone where the gas is more concentrated, said zone accordingly being designated "riser", and in contrast to descend in the zone with a lower dispersed gas content, said zone accordingly being designated "downcomer".

The gas stream may also follow a different path, as illustrated in Figure 2. In this case, the gas is distributed outside the draft tube and the interspace between the latter and the walls of the reaction device accordingly acts as a riser, while the draft tube acts as a downcomer.

With reference to Figure 3, a draft tube is a tubular element with a variable diameter along the axis thereof. The upper part of the draft tube (4) may have a cylindrical shape and diameter D2, the intermediate part may have a truncated cone shape, while the lower part may have a cylindrical shape of diameter D1.

A further embodiment of the present invention is a fed-batch aerobic fermentation process which is carried out in the air-lift reaction device described and claimed in the present text.

The fed-batch method provides that a fermentation reaction is carried out in two successive stages in the same reaction device. In the first reaction stage, the reaction is started in discontinuous or batch mode until a reactant is partially or completely consumed; in the case of a fermentation, it is the substrate which is consumed. In the second reaction stage, the consumed reactant is continuously fed in at a flow rate such as to maintain an optimum concentration in the reactor. Feeding of the consumed reactant leads to an increase in reaction volume. Only air is fed in continuously mode during both stages.

The fed-batch mode permits better management of the biochemical reactions for which the reaction rates are a function of the metabolism of the cellular types in question. For example, a high substrate concentration in the initial (batch) stage of the reaction allows rapid cellular reproduction; a subsequent reduction and maintenance of the substrate (continuous substrate feed) makes it possible to extend the steady-state stage to obtain the final product ("Biochemical Engineering Fundamentals"; J.E. Bailey, D.F. Ollis; McGraw-Hill; second edition; 1986).

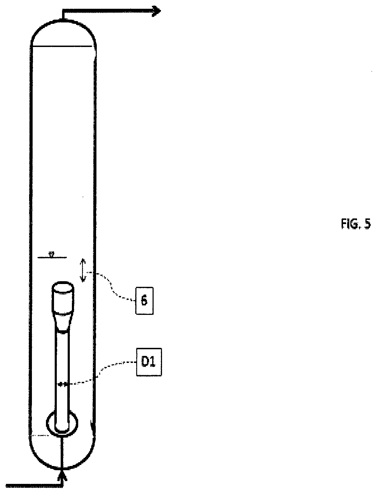

The process provided by the present invention includes therefore the following stages:
a)at least partially filling the reaction device described and claimed in the present text with a liquid-phase reactant; this stage constitutes the preparation of the batch method as illustrated in Figure 5 (6);
b)feeding a gas-phase reactant into said reaction device so as that a liquid-phase reaction occurs until the reactants have been at least partially consumed; this stage constitutes the batch reaction stage;
c)feeding into said reaction device a liquid stream containing the liquid-phase reactants consumed during the batch stage and the gas-phase reactant; this stage constitutes the continuous reaction stage;
d)discontinuing feed of both the liquid-phase reactant and the gas-phase reactant once the desired amount of product has been obtained.

The liquid-phase reactants introduced in stage (a) are those necessary for the reaction for the duration of the batch stage (b) alone.

The liquid-phase reactant is not fed in during the batch mode reaction (b).

The flow rate of the liquid stream fed in in stage (c) depends on the rate of consumption of the liquid-phase reactants. Gas is constantly fed into the reactor during this stage.

### Inventive example

An aerobic fermentation for producing oleaginous yeasts was carried out in an air-lift reactor as described and claimed in the present text and having the following characteristics:
- a single draft tube,
- geometry of the draft tube: D1=0.8 m, D2=1.3 m, total height 4.0 m, height of section with diameter D2 equal to 0.6 m, height of truncated cone equal to 0.8 m,
- geometry of the cylindrical volume: internal diameter equal to 2.0 m with a height of 9.5 m,
- air distributor outside the draft tube,

The fermentation was carried out in fed-batch mode according to the following stages:
a)preparation in batch stage; the reactor was charged with 13.5 m³ of aqueous solution containing glucose (liquid-phase reactant) at a concentration of 56 to 111 g/L;
b)batch reaction stage; steps (i), (ii) and (iii) described below were performed in this stage.
c)continuous reaction stage: steps (iv) and (v) described below were carried out in this stage.
d)end of the reaction; once the desired amount of product, oleaginous yeasts in a concentration equal to 100 g/L, had been obtained, feed of both liquid to the reactor and air was discontinued.

The final volume of liquid will be approx. 30 m³, with a duration of fermentation of 96 h. The energy consumption required for the complete fermentation was 5.6 MWh.

### Step (i).

Air (the oxygen in the air is the gas-phase reactant) was fed into the reactor at flow rate equal to 600 Nm³/h until the liquid was saturated with oxygen. The concentration of oxygen in the liquid was equal to 8.0e-3 g/L at 30°C and 1.4 bara.

### Step (ii).

An inoculum of 1.5 m³ of liquid containing oleaginous yeasts (reaction product) at a concentration of approx. 20 g/L was fed into the reactor. Addition of the inoculum to the reactor indicated the start of the oleaginous yeast growth reaction with consequent consumption of fed oxygen and the glucose present in the liquid.

### Step (iii).

Air was fed into the reactor to keep the concentration of dissolved oxygen in the liquid greater than 2.6e-3 g/L. The demanded air flow rate grew constantly over time. The maximum air flow rate required to sustain the reaction was equal to 2900 Nm³/h, corresponding to a consumed (compressor) power equal to 173 kW. A reduction in demanded air flow rate indicated a shortage of glucose and hence the end of the batch stage. The batch stage had a duration of approx. 24 h from the start of the reaction.

### Step (iv).

An aqueous solution containing glucose at a concentration of 600 g/L was continuously fed into the reactor. The flow rate of said stream had to be such as to maintain a glucose concentration of approx. 30 g/L in the reactor. Said flow rate proved to be approx.
0.2 m³/h.

### Step (v)

Air was fed into the reactor to keep the concentration of dissolved oxygen in the liquid greater than 3.4e-3 g/L. The air flow rate varied continuously over the course of the stage between 2900 and 800 Nm³/h.

### Comparative example 1

An aerobic fermentation for producing oleaginous yeasts was carried out in a bubble column reactor having the following characteristics:
- geometry of the reaction device: internal diameter equal to 2.0 m with a height of 9.5 m,
- air distributor at the base of the device.

Fermentation was carried out in fed-batch mode following the same stages as the inventive example:
a)preparation in batch stage; the reactor was charged with 13.5 m³ of aqueous solution containing glucose (liquid-phase reactant) at a concentration of 56 to 111 g/L;
b)batch reaction stage; steps (I), (II) and (III) were performed in this stage;
c)continuous reaction stage; the following steps (IV) and (IV) were performed in this stage;
d)end of the reaction; once the desired amount of product, oleaginous yeasts in a concentration equal to 100 g/L, had been obtained, feed of both liquid to the reactor and air was discontinued.

The final volume of liquid will be approx. 30 m³, with a duration of fermentation of 96 h. The estimated energy consumption required for the complete fermentation was 7.2 MWh.

### Step (I)

Air (the oxygen in the air is the gas-phase reactant) was fed into the reactor at a flow rate equal to 600 Nm³/h until the liquid was saturated with oxygen, the concentration of oxygen in the liquid being equal to 8.0e-3 g/L at 30°C and 1.4 bara.

### Step (II)

An inoculum of 1.5 m³ of liquid containing oleaginous yeasts (reaction product) at a concentration of approx. 20 g/L was fed into the reactor. Addition of the inoculum to the reactor indicated the start of the oleaginous yeast growth reaction with consequent consumption of fed oxygen and the glucose present in the liquid.

### Step (III)

Air was fed into the reactor to keep the concentration of dissolved oxygen in the liquid greater than 2.6e-3 g/L. The demanded air flow rate grew constantly over time. The maximum air flow rate required to sustain the reaction was equal to 3400 Nm³/h, corresponding to a consumed (compressor) power equal to 200 kW. A reduction in demanded air flow rate indicated a shortage of glucose and hence the end of the batch stage. The batch stage had a duration of approx. 24 h from the start of the reaction.

### Step (IV)

An aqueous solution containing glucose at a concentration of 600 g/L was continuously fed into the reactor. The flow rate of said stream had to be such as to maintain a glucose concentration of approx. 30 g/L in the reactor. Said flow rate proved to be approx.
0.2 m³/h.

### Step (V)

Air was fed into the reactor to keep the concentration of dissolved oxygen in the liquid greater than 3.5e-3 g/L. The air flow rate varied continuously over the course of the stage between 3400 and 1000 Nm³/h.

## Claims

1. A reaction device with air-lift type internal circulation which comprises:
- a vertical cylindrical volume (1);
- a vertical element (2) positioned within said volume in such a way as to form an interspace with the walls of said volume, having a cross-section which is circular and orthogonal to the vertical axis of the element and with a variable internal diameter along said axis, said element being denoted "draft tube";
- at least one gas distributor (3) positioned on the bottom of said device;
said device being **characterised in that**:
- the ratio between the diameter of the internal vertical element (2) and the internal diameter of the cylindrical volume (1) ranges from 0.05 to 0.5, and
- the ratio between the height of the vertical element (2) and the height of the cylindrical volume (1) is less than 0.5.

2. A device according to claim 1, in which the vertical element inside the reaction device is formed by three bodies, two cylindrical bodies having different diameters (2, 4) and a body in the form of a truncated cone (5), said bodies being connected to one another in such a manner that the minor base of the truncated cone (5) coincides with one of the bases of the first cylindrical body (2) and the major base of the truncated cone coincides with one of the bases of the second cylindrical body (4).

3. A device according to claim 2, in which the ratio between the height of the truncated cone in the draft tube and the total height of the device ranges from 0.03 to 0.25.

4. A device according to claim 2, in which the inclination of the apothem of the truncated cone body in the draft tube ranges from 15° to 30°.

5. A device according to any of claims 1 to 4, in which the ratio between the diameter of the internal vertical element and the internal diameter of the cylindrical volume ranges from 0.35 to 0.45.

6. A device according to any one of claims 1 to 5, in which the ratio between the height of the vertical element (2) and the height of the cylindrical volume (1) is less than 0.5 and greater than 0.25.

7. A fed-batch aerobic fermentation process which is carried out in the air-lift reaction device according to any one of claims 1 to 6 and comprises the following stages:
a)at least partially filling said reaction device with a liquid-phase reactant;
b)feeding a gas-phase reactant into said reaction device in batch mode so as to bring about a liquid-phase reaction until the reactants have been at least partially consumed;
c)feeding a liquid stream containing the liquid-phase reactants consumed during stage (b) into said reaction device in batch mode while feeding the gas-phase reactant in continuous mode;
d)discontinuing feed of both the liquid-phase reactant and the gas-phase reactant once the desired amount of product has been obtained.

## Patentansprüche

1. Eine Reaktionsvorrichtung mit interner Luftumwälzung vom *Airlift*-Typ, die Folgendes umfasst:
- ein vertikales zylindrisches Volumen (1);
- ein vertikales Element (2), das im Inneren des genannten Volumens so angeordnet ist, dass es einen Zwischenraum mit den Wänden des genannten Volumens bildet, und das einen kreisförmigen Querschnitt orthogonal zur vertikalen Achse des Elements und einen variablen Innendurchmesser entlang der genannten Achse aufweist, wobei das genannte Element als "Saugrohr" *("draft tube")* bezeichnet wird;
- mindestens einen Gasverteiler (3), der auf dem Boden der genannten Vorrichtung angeordnet ist;
wobei die genannte Vorrichtung **dadurch gekennzeichnet ist, dass:**
- das Verhältnis zwischen dem Durchmesser des inneren vertikalen Elements (2) und dem Innendurchmesser des zylindrischen Volumens (1) zwischen 0,05 und 0,5 liegt, und
- das Verhältnis zwischen der Höhe des vertikalen Elements (2) und der Höhe des zylindrischen Volumens (1) kleiner als 0,5 ist.

2. Eine Vorrichtung nach Anspruch 1, bei der das vertikale Element im Inneren der Reaktionsvorrichtung aus drei Körpern gebildet ist, zwei zylindrischen Körpern mit unterschiedlichen Durchmessern (2, 4) und einem Körper in Form eines Kegelstumpfes (5), wobei die genannten Körper so miteinander verbunden sind, dass die kleinere Basis des Kegelstumpfes (5) mit einer der Basen des ersten zylindrischen Körpers (2) und die größere Basis des Kegelstumpfes mit einer der Basen des zweiten zylindrischen Körpers (4) zusammenfällt.

3. Eine Vorrichtung nach Anspruch 2, bei der das Verhältnis zwischen der Höhe des Kegelstumpfes im Saugrohr und der Gesamthöhe der Vorrichtung zwischen 0,03 und 0,25 liegt.

4. Eine Vorrichtung nach Anspruch 2, bei der die Neigung des Apothemas des Kegelstumpfkörpers im Saugrohr zwischen 15° und 30° liegt.

5. Eine Vorrichtung nach irgendeinem der Ansprüche von 1 bis 4, bei der das Verhältnis zwischen dem Durchmesser des inneren vertikalen Elements und dem Innendurchmesser des zylindrischen Volumens zwischen 0,35 und 0,45 liegt.

6. Eine Vorrichtung nach irgendeinem der Ansprüche von 1 bis 5, bei der das Verhältnis zwischen der Höhe des vertikalen Elements (2) und der Höhe des zylindrischen Volumens (1) kleiner als 0,5 und größer als 0,25 ist.

7. Ein aerobes *Fed-Batch*-Fermentationsverfahren, das in der *Airlift-*Reaktionsvorrichtung nach irgendeinem der Ansprüche von 1 bis 6 durchgeführt wird und die folgenden Schritte umfasst:
a) zumindest teilweises Befüllen der genannten Reaktionsvorrichtung mit einem Flüssigphasen-Reaktanten;
b) Zuführen eines Gasphasen-Reaktanten in die genannte Reaktionsvorrichtung im Chargenbetrieb (*batch mode*), um eine Flüssigphasenreaktion herbeizuführen, bis die Reaktanten zumindest teilweise verbraucht worden sind;
c) Zuführen eines Flüssigkeitsstroms, der die im Schritt (b) verbrauchten Flüssigphasen-Reaktanten enthält, in die genannte Reaktionsvorrichtung im Chargenbetrieb, während der Gasphasen-Reaktant im kontinuierlichen Modus eingespeist wird;
d) Unterbrechen der Zufuhr sowohl des Flüssigphasen-Reaktanten als auch des Gasphasen-Reaktanten, sobald die gewünschte Produktmenge erreicht ist.

## Revendications

1. Dispositif de réaction présentant une circulation interne de type à agitation pneumatique qui comprend :
- un volume cylindrique vertical (1) ;
- un élément vertical (2) positionné à l'intérieur dudit volume de manière à former un espace intermédiaire avec les parois dudit volume, ayant une section transversale qui est circulaire et orthogonale à l'axe vertical de l'élément et présentant un diamètre interne variable le long dudit axe, ledit élément étant appelé « tube de tirage » ;
- au moins un distributeur de gaz (3) positionné sur le fond dudit dispositif ;
ledit dispositif étant **caractérisé en ce que** :
- le rapport entre le diamètre de l'élément vertical interne (2) et le diamètre interne du volume cylindrique (1) se situe dans une plage allant de 0,05 à 0,5, et
- le rapport entre la hauteur de l'élément vertical (2) et la hauteur du volume cylindrique (1) est inférieur à 0,5.

2. Dispositif selon la revendication 1, dans lequel l'élément vertical à l'intérieur du dispositif de réaction est formé par trois corps, deux corps cylindriques ayant différents diamètres (2, 4) et un corps sous la forme d'un cône tronqué (5), lesdits corps étant connectés les uns aux autres de telle manière que la petite base du cône tronqué (5) coïncide avec l'une des bases du premier corps cylindrique (2) et la grande base du cône tronqué coïncide avec l'une des bases du second corps cylindrique (4).

3. Dispositif selon la revendication 2, dans lequel le rapport entre la hauteur du cône tronqué dans le tube de tirage et la hauteur totale du dispositif se situe dans une plage allant de 0,03 à 0,25.

4. Dispositif selon la revendication 2, dans lequel l'inclinaison de l'apothème du corps sous forme de cône tronqué dans le tube de tirage se situe dans une plage allant de 15° à 30°.

5. Dispositif selon l'une quelconque des revendications 1 à 4, dans lequel le rapport entre le diamètre de l'élément vertical interne et le diamètre interne du volume cylindrique se situe dans une plage allant de 0,35 à 0,45.

6. Dispositif selon l'une quelconque des revendications 1 à 5, dans lequel le rapport entre la hauteur de l'élément vertical (2) et la hauteur du volume cylindrique (1) est inférieur à 0,5 et supérieur à 0,25.

7. Procédé de fermentation aérobie à alimentation discontinue qui est réalisé dans le dispositif de réaction à agitation pneumatique selon l'une quelconque des revendications 1 à 6 et comprend les étapes suivantes :
a) le remplissage au moins partiel dudit dispositif de réaction avec un réactif en phase liquide ;
b) l'introduction d'un réactif en phase gazeuse dans ledit dispositif de réaction en mode discontinu de façon à provoquer une réaction en phase liquide jusqu'à ce que les réactifs aient été au moins partiellement consommés ;
c) l'introduction d'un flux liquide contenant les réactifs en phase liquide consommés pendant l'étape (b) dans ledit dispositif de réaction en mode discontinu tout en introduisant le réactif en phase gazeuse en mode continu ;
d) l'interruption de l'introduction à la fois du réactif en phase liquide et du réactif en phase gazeuse une fois que la quantité souhaitée de produit a été obtenue.
